# FASCICULE DE BREVET EUROPEEN

(11) **EP 1 929 975 B1**
(45) Date de publication et mention de la délivrance du brevet: **19.10.2011**
(21) Numéro de dépôt: 07122428.1
(22) Date de dépôt: 05.12.2007
(51) Int. Cl.: A61C 8/00, A61B 17/88

(54) **Tournevis à usage dentaire ou chirurgical**
Schraubenzieher für den Einsatz im zahnmedizinischen oder chirurgischen Bereich
Screwdriver for dental or surgical use

(30) Priorité: 07.12.2006 CH 19972006; 11.12.2006 CH 20122006; 14.06.2007 CH 9592007
(43) Date de publication de la demande: 11.06.2008
(73) Titulaire: Bien-Air Holding SA, 2504 Bienne (CH)
(72) Inventeur: Mosimann, David, 2503, Bienne (CH)
(74) Mandataire: Ravenel, Thierry Gérard Louis

(56) Documents cités:
- WO-A-93/20773
- WO-A-2006/133973
- US-A- 5 139 499
- US-A1- 2005 033 307

## Description

La présente invention concerne un tournevis à usage dentaire ou chirurgical. Plus précisément, l'invention concerne un tournevis à usage dentaire ou chirurgical d'une utilisation simple et permettant de visser plus facilement des vis par exemple dans la bouche d'un patient pour l'installation d'une prothèse ou dans un os pour la réduction d'une fracture.

Les personnes à qui manque une dent, une grande partie d'entre elles ou toutes les dents sont classiquement équipées d'une prothèse amovible ou d'un dentier. Un tel dentier se compose classiquement de dents en résine fixées sur un socle lui-même en résine qui épouse la forme de la gencive. La moitié supérieure du dentier comprend en outre une paroi qui épouse le profil du palais de la bouche du porteur, ceci afin d'augmenter les forces de capillarité entre ledit dentier et le palais et éviter les risques de chute du dentier supérieur. Certaines personnes, pour améliorer plus encore les forces d'adhésion entre leur dentier et la gencive, utilisent un adhésif temporaire qu'elles déposent sur les surfaces du dentier destinées à venir en contact avec leurs gencives.

Malgré tout le soin avec lequel les prothésistes dentaires et les dentistes réalisent les dentiers, et malgré l'utilisation d'adhésifs, force est de reconnaître que les dentiers ne tiennent pas très bien en bouche. Ceci est particulièrement vrai lorsque la personne vieillit et que sa gencive se rétracte. La géométrie du dentier n'est alors plus parfaitement adaptée au profil de la gencive et il apparaît des jeux entre ledit dentier et la gencive. La seule solution consiste alors à reprendre le dentier ou à en faire fabriquer un nouveau, ce qui est coûteux.

Pour remédier aux problèmes mentionnés ci-dessus, une solution consiste à équiper le patient d'un dentier que le dentiste fixe de manière amovible dans la bouche du patient. Une telle solution est beaucoup plus confortable pour le patient qui recouvre une dentition quasi semblable à sa dentition naturelle et des facultés de mastication optimales. Pour atteindre ce but, le dentiste va installer des implants dans l'os maxillaire supérieur et/ou inférieur de son patient. Pour chacune des mâchoires inférieure et supérieure, il est prévu des implants du type bouton-pression à visser dans l'os. On en dispose habituellement deux premiers à environ deux centimètres de part et d'autre de la ligne médiane de la mâchoire et deux autres plus en arrière dans la bouche à environ un centimètre des deux premiers. Une fois les implants consolidés, étape qui dure habituellement environ six mois, le dentiste va poser le dentier sur la gencive du patient, puis l'immobiliser au moyen des boutons-pression maintenus par ancrage sphérique dans des cavaliers eux-mêmes vissés dans des trous filetés correspondants prévus dans les implants.

Durant toute la phase de consolidation, les trous filetés des implants sont obturés au moyen de vis de cicatrisation de petites dimensions. De telles vis sont difficiles à mettre en place, non seulement en raison de leurs faibles dimensions, mais également en raison du fait que les implants installés dans la bouche du patient ne sont pas faciles d'accès. Pour mener à bien ce travail, le dentiste dispose d'un tournevis aux dimensions appropriées dont l'extrémité de la tige présente un profil correspondant au profil de l'empreinte pratiquée dans la tête de la vis. Le dentiste place la vis à l'extrémité du tournevis et tout en maintenant l'ensemble en équilibre, tente de placer la vis sur son implant jusqu'à ce que les filets de la vis viennent en prise avec le filetage du trou pratiqué dans l'implant. On comprend donc que ces opérations de pose de la vis de cicatrisation dans un implant dentaire sont malcommodes, notamment en raison des difficultés pour accéder à la bouche du patient, et qu'elles exigent du temps et de la dextérité de la part du dentiste. Il n'est néanmoins pas rare que le dentiste perde la vis et que celle-ci soit ingérée par le patient. Dans des cas graves, il peut même arriver que la vis s'engage dans le canal pulmonaire du patient.

Pour faciliter le travail du dentiste, il est prévu de réaliser le tournevis et les vis avec des tolérances sévères, de façon que lorsqu'on engage le tournevis dans la tête creuse de la vis, celle-ci se maintienne au bout de la tige du tournevis. En raison des tolérances avec lesquelles ils doivent être fabriqués, de tels tournevis sont coûteux à produire et présentent donc un prix de vente élevé. De plus, de tels tournevis sont à usage quasi unique car après que le dentiste s'en soit servi pour visser quelques vis, des jeux apparaissent entre la vis et l'extrémité de la tige du tournevis et le dentiste ne parvient plus à maintenir lesdites vis en équilibre au bout de son tournevis.

Le document US5139499 divulgue un tournevis chirurgical selon le préambule de la revendication 1.

La présente invention a pour but de remédier aux inconvénients susmentionnés ainsi qu'à d'autres encore en procurant un tournevis à usage dentaire ou chirurgical qui simplifie la tâche du dentiste lors du vissage d'une vis dans la bouche d'un patient.

A cet effet, la présente invention concerne un tournevis à usage dentaire ou chirurgical s'étendant sensiblement rectilignement selon un axe de symétrie longitudinale et comprenant du côté proximal un manche par lequel le praticien tient le tournevis, ce manche étant prolongé du côté distal par une tige qui présente à son extrémité libre un profil correspondant au profil d'une empreinte pratiquée dans la tête d'une vis à visser, ce tournevis étant **caractérisé en ce que** l'extrémité libre de la tige est séparée par au moins une fente en au moins deux parties présentant une élasticité et en ce qu'il comprend des moyens de contrôle de l'écartement montés fixes entre les deux parties élastiques qui empêchent ces deux parties élastiques de se rapprocher l'une de l'autre lorsqu'on exerce un couple de serrage au moyen du tournevis.

Grâce à ces caractéristiques, la présente invention procure un tournevis à usage dentaire ou chirurgical qui permet de retenir de manière efficace la vis au bout de la tige du tournevis. Le dentiste ou le chirurgien peut donc, à l'aide de son tournevis, amener la vis en regard de l'implant et commencer à engager les filets de la vis avec le filetage du trou pratiqué dans l'implant, ce qui facilite considérablement son travail.

Ce résultat avantageux est atteint grâce au fait que l'extrémité de la tige est fendue en au moins deux parties légèrement écartées l'une de l'autre par élasticité. De la sorte, lorsqu'on introduit l'extrémité de la tige du tournevis dans l'empreinte pratiquée dans la tête de la vis, les deux parties de cette tige délimitées par la fente ont tendance à se resserrer l'une contre l'autre, exerçant ainsi une force de rappel élastique contre les rebords de l'empreinte de la vis dans laquelle elles sont engagées. On parvient ainsi à maintenir efficacement la vis à l'extrémité de la tige du tournevis, de sorte qu'il est plus facile pour le dentiste de positionner convenablement ladite vis en regard de l'implant ou pour un chirurgien en regard de l'avant trou de l'os et de commencer à visser.

En outre, la présence des moyens de contrôle de l'écartement entre les deux parties élastiques délimitées par la fente empêche ces deux parties élastiques de se rapprocher l'une de l'autre et permet d'éviter qu'elles ne se tordent, voire cassent lorsqu'on exerce un couple de serrage au moyen du tournevis selon l'invention.

On a en effet constaté que l'inconvénient des tournevis dont le bout de la lame est fendu est que les deux moitiés fendues ont tendance, en raison de la présence de la fente et du manque de matière correspondant, à se rapprocher l'une de l'autre sous l'effet du couple de serrage et à se tordre axialement. Par conséquent, il peut arriver que le tournevis n'ait plus prise sur l'empreinte de la vis et tourne dans le vide dans certains cas.

Une solution pour remédier à ce problème consisterait à rendre la fente pratiquée dans le bout de la lame du tournevis aussi mince que possible. Ceci est néanmoins très complexe à réaliser et donc très coûteux en termes d'usinage.

Ainsi, les moyens selon l'invention, sans contrevenir à l'élasticité des parties terminales de la tige du tournevis, permettent cependant d'empêcher ces parties terminales de se tordre en réduisant sensiblement le jour entre elles et ce pour un prix de revient raisonnable.

Un autre avantage de l'invention réside dans le fait que les tournevis peuvent être fabriqués selon des tolérances normalisées moins sévères que par le passé. De tels tournevis sont donc moins coûteux à l'achat et peuvent en outre servir plus longtemps que les tournevis de l'art antérieur dans la mesure où le maintien de la vis au bout du tournevis repose sur des phénomènes de déformations élastiques qui sont moins sujets à des problèmes de jeu.

Selon une première variante de réalisation, les moyens qui empêchent les deux parties élastiques terminales de la tige du tournevis de se rapprocher l'une de l'autre comprennent une goupille fixée dans un alésage pratiqué axialement à l'intérieur de la tige du tournevis, à partir de l'extrémité libre de celle-ci.

La goupille est rendue solidaire de la tige du tournevis par chassage, collage ou soudage.

Selon une seconde variante de réalisation, le tournevis comprend d'une part un tube creux présentant une extrémité proximale et une extrémité distale, l'extrémité distale du tube présentant un profil correspondant au profil d'une empreinte pratiquée dans la tête d'une vis à visser et étant séparée par au moins une fente en au moins deux parties présentant une élasticité et, d'autre part, un cylindre introduit coaxialement à l'intérieur du tube creux et s'étendant sensiblement jusqu'au niveau de l'extrémité distale de ce tube, le cylindre étant solidaire du tube creux et se prolongeant du côté proximal par un manche.

Le cylindre peut être un tube creux ou un tube plein. II est rendu solidaire du tube creux à l'intérieur duquel il est introduit par collage ou soudage.

Les deux variantes de réalisation mentionnées ci-dessus permettent tout à la fois d'empêcher que les deux moitiés de tube ne se resserrent de trop et d'avoir des fentes d'une largeur plus facilement usinable.

Selon une caractéristique complémentaire de l'invention, l'extrémité de la tige du tournevis est fendue diamétralement.

Selon cette autre caractéristique, les deux parties de la tige du tournevis délimitées par la fente sont symétriques. Les deux parties de la tige présentent ainsi une même résistance mécanique à la torsion lorsqu'on exerce sur la vis un couple de serrage au moyen du tournevis. De même, ces deux parties exercent sur la vis la même force de rappel élastique, ce qui permet d'équilibrer au mieux les forces qui permettent de retenir la vis sur l'extrémité de la tige du tournevis.

Selon encore une autre caractéristique, l'extrémité de la tige du tournevis est fendue diamétralement selon au moins deux fentes.

D'autres caractéristiques et avantages de la présente invention ressortiront plus clairement de la description détaillée qui suit de différents modes de réalisation du tournevis selon l'invention, ces exemples étant donnés à titre purement illustratif et non limitatif seulement en liaison avec le dessin annexé sur lequel:

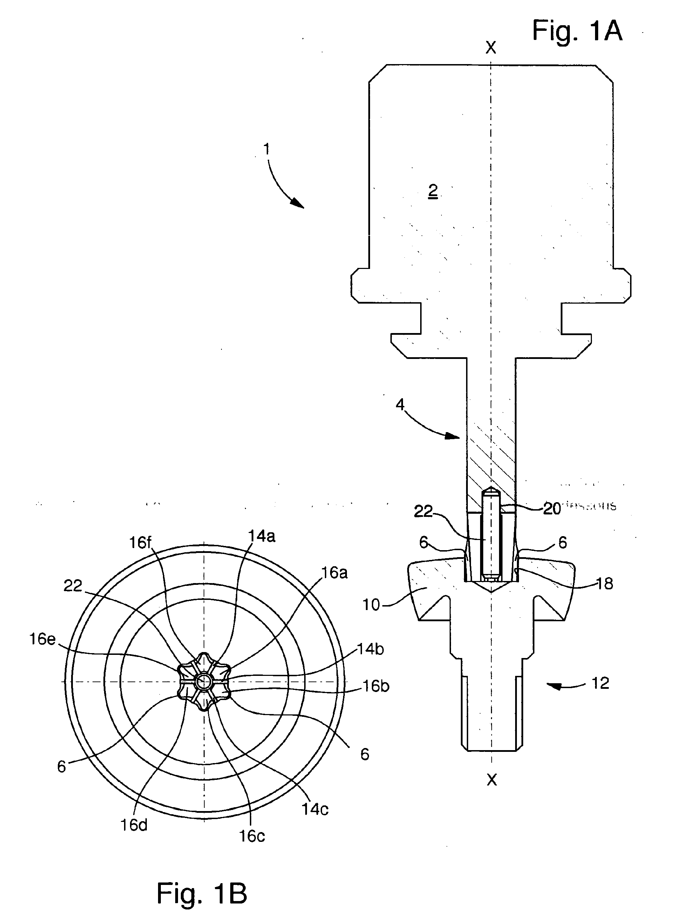

- les figures 1A et 1B sont respectivement des vues latérale et d'extrémité d'un tournevis à usage dentaire ou chirurgical selon un premier mode de réalisation de l'invention, l'extrémité libre de la tige du tournevis étant diamétralement fendue selon trois fentes géométriquement espacées avec une goupille chassée dans un perçage pratiqué dans ladite tige;

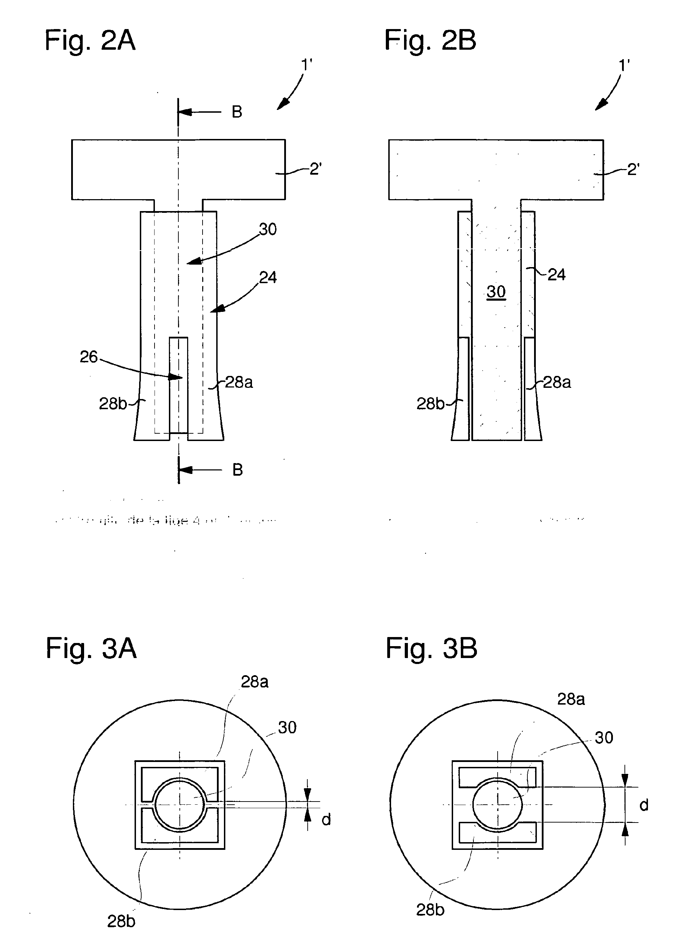

- les figures 2A et 2B sont respectivement des vues latérale et en coupe longitudinale d'un tournevis à usage dentaire ou chirurgical selon un second mode de réalisation de l'invention, le tournevis se composant d'un tube creux fendu à l'intérieur duquel est introduit un tube plein prolongé par un manche, et
- les figures 3A et 3B sont des vues d'extrémité de deux tournevis selon l'invention permettant d'illustrer la possibilité offerte par l'invention de contrôler la largeur de la fente du tournevis.

La présente invention procède de l'idée générale inventive qui consiste à séparer par au moins une fente l'extrémité libre de la tige d'un tournevis en au moins deux parties présentant une élasticité de façon à pouvoir passer d'une première position de repos dans laquelle elles sont détendues à une seconde position comprimée dans laquelle elles exercent une force de rappel élastique sur les rebords de l'empreinte prévue dans la tête d'une vis à visser. Grâce à cela, il est possible de retenir la vis au bout de la tige du tournevis, ce qui simplifie considérablement le travail du dentiste ou du chirurgien qui peut approcher la vis de l'implant ou de l'avant trou pratiqué dans un os dans lequel cette vis doit être vissée au moyen du tournevis. La présente invention se révèle particulièrement avantageuse dans les cas où le dentiste doit visser des vis dans des endroits difficiles d'accès, notamment aux extrémités postérieures de la gencive. La présente invention prévoit en outre des moyens pour contrôler l'écartement entre les deux parties élastiques et empêcher ces deux parties élastiques de se rapprocher l'une de l'autre lorsqu'on exerce un couple de serrage au moyen du tournevis. Grâce à cette autre caractéristique, on comble l'espace laissé vacant par la fente entre les deux parties élastiques et on empêche tout phénomène de cisaillement des parties élastiques. Le risque que les parties élastiques cassent sous l'effet du couple exercé et que le tournevis tourne à vide dans l'empreinte de la vis est donc évité. Surtout, cette disposition permet d'éviter de devoir réaliser une fente très mince afin de limiter l'espace laissé vacant entre les deux parties élastiques, ce qui permet de limiter le prix de revient d'un tournevis selon l'invention.

La présente invention va notamment être décrite en liaison avec un tournevis et une vis du type à six lobes encore connu sous le nom de "principe Torx". Autrement dit, l'extrémité libre de la tige du tournevis comprend six lobes régulièrement espacés et la tête de la vis comprend une empreinte en forme d'étoile à six branches destinée à recevoir l'extrémité libre de la tige du tournevis. Il va néanmoins de soi que cet exemple est donné à titre purement illustratif seulement et que la présente invention s'applique à tout type de vis à tête creuse et aux tournevis correspondants. Ainsi, il peut être envisagé d'appliquer l'invention de manière identique à un tournevis répondant au standard "Imbus" et présentant à cet effet une tige conformée en hexagone à son extrémité libre ou bien à un tournevis à profil carré.

La figure 1A est une vue latérale d'un tournevis selon un premier mode de réalisation de l'invention. Comme il ressort de cette figure, le tournevis, désigné dans son ensemble par la référence numérique générale 1, comprend du côté proximal un manche 2 de forme sensiblement cylindrique que le dentiste tient typiquement entre deux de ses doigts. Du côté distal, le tournevis 1 est prolongé par une tige 4 qui s'étend rectilignement selon l'axe de symétrie longitudinale X-X dudit tournevis 1. Le tournevis 1 représenté à la figure 1A est un tournevis qui répond au standard "Torx" ou six lobes. Autrement dit, sa tige 4 présente à son extrémité libre six lobes 6 (deux d'entre eux seulement sont visibles à la figure 1A, les six étant visibles de dessous sur la figure 1 B) qui sont régulièrement espacés le long de la circonférence de ladite tige 4. Ces six lobes 6 sont destinés à être reçus dans une empreinte 8 en forme d'étoile à six branches pratiquée dans la tête 10 d'une vis 12. On rappelle à nouveau que la présente invention n'est pas limitée à un tel type d'ensemble tournevis/vis et qu'elle s'applique indifféremment à tout type de tournevis à usage dentaire ou chirurgical destiné au vissage d'une vis à tête creuse.

Conformément à la présente invention, on réalise de bas en haut au moins une, et par exemple trois fentes 14a-14c dans l'extrémité libre de la tige 4 du tournevis 1. Ces fentes peuvent être réalisées au moyen d'un disque de fraisage ou au fil. Comme on le voit clairement sur la figure 1B, ces trois fentes diamétrales 14a-14c sont à chaque fois situées entre deux lobes 6 successifs du tournevis 1 et partagent l'extrémité de la tige 4 en six parties 16a-16f identiques légèrement écartées les unes des autres par élasticité. L'élasticité peut par exemple être obtenue en chassant légèrement un coin dans l'extrémité libre de la tige 4, ce qui va avoir pour effet d'écarter les unes des autres les parties élastiques et leur conférer l'élasticité requise. Il est également envisageable d'écarter les parties élastiques les unes des autres au moyen d'une pince.

Ainsi, lorsque le tournevis 1 est engagé dans la tête creuse de la vis, ces parties élastiques 16a-16c se rapprochent les unes des autres et exercent une force de contre-réaction élastique sur les bords 18 de l'empreinte 8. Il est donc possible de retenir la vis 12 au bout du tournevis 1, ce qui facilite considérablement le travail du dentiste qui peut approcher la vis 12 de l'implant (non représenté) dans lequel elle doit être vissée au moyen du tournevis 1. Le tournevis 1 peut alors être utilisé à la façon d'un prolongateur permettant au dentiste d'atteindre les endroits de la bouche du patient difficiles d'accès.

En augmentant le nombre de fentes, on augmente l'élasticité de l'extrémité de la tige 4 du tournevis 1 et donc les forces de rappel élastiques qui s'exercent sur la vis 12 lorsque ledit tournevis 1 est engagé dans la tête creuse 10 de ladite vis 12. Comme on pourra le constater à l'examen des figures 1A et 1B, les fentes 14a-14c sont pratiquées selon des diamètres de la tige 4, ceci dans le but de la partager en parties identiques 16a-16f qui présenteront le même comportement élastique vis-à-vis du couple de serrage exercé par le praticien sur le tournevis 1. Il va néanmoins de soi que dans une variante simplifiée d'exécution de l'invention, les fentes 14 n'ont pas impérativement besoin d'être pratiquées avec une telle précision et peuvent être décalées par rapport au plan de symétrie axiale de la tige 4.

Conformément à une autre caractéristique de l'invention, on pratique dans l'extrémité de la tige 4 du tournevis 1 un perçage 20 aligné sur l'axe de symétrie longitudinale X-X du tournevis 1 et dans lequel on introduit ensuite une goupille 22. Cette goupille 22 a pour but de combler le vide laissé vacant par les fentes 14a-14c entre les six parties terminales 16a-16f de la tige 4 du tournevis 1. On a en effet constaté qu'en l'absence de la goupille 22, les parties 16a-16f de la tige 4 du tournevis 1 délimitées par les fentes 14a-14c ont tendance à se tordre, voire à casser, lorsqu'on exerce un couple de serrage au moyen dudit tournevis 1. Ceci peut en effet survenir en raison du manque de matière entre ces différentes parties 16a-16f, manque qui autorise ces parties à se déplacer lorsqu'on exerce un couple de serrage au moyen du tournevis 1. En introduisant une goupille 22 dans le perçage 20 concentriquement à la tige 4 du tournevis 1, les parties terminales 16a-16f de la tige 4 du tournevis 1 délimitées par les fentes 14a-14c conservent leur élasticité mais voient leur liberté de se rapprocher les unes des autres quasi annulée. Les risques qu'elles soient soumises à un phénomène de cisaillement et qu'elles cassent sont dont quasiment annulés. La goupille 22 peut être chassée dans le perçage 20. Elle peut aussi être collée ou soudée.

Un second mode de réalisation de l'invention est illustré en liaison avec les figures 2A et 2B. Conformément à ce second mode de réalisation, le tournevis, désigné dans son ensemble par la référence numérique générale 1', comprend un tube creux 24 présentant une extrémité proximale et une extrémité distale, l'extrémité distale du tube 24 présentant un profil par exemple carré correspondant au profil d'une empreinte pratiquée dans la tête d'une vis à visser et étant séparée par au moins une fente 26 en au moins deux parties 28a, 28b présentant une élasticité. Comme précédemment, l'élasticité peut être obtenue en chassant légèrement un coin dans l'extrémité distale du tube 24 ou en écartant les deux parties élastiques 28a, 28b l'une de l'autre au moyen d'une pince. Par ailleurs, il va de soi que le nombre de fentes pratiquées dans l'extrémité distale du tube 24 n'est pas limité à un et peut être, par exemple, de deux ou trois. Préférentiellement mais non impérativement, la ou les fentes 26 sont réalisées selon des diamètres du tube creux 24.

Le tournevis 1' conforme au second mode de réalisation de l'invention est complété par un cylindre 30 introduit coaxialement à l'intérieur du tube creux 24 du côté de l'extrémité proximale de celui-ci et s'étendant sensiblement jusqu'au niveau de l'extrémité distale de ce tube 24 ou affleurant avec cette extrémité distale. Ce cylindre 30 peut être lui-même un tube creux ou bien être un tube plein. Il se prolonge du côté proximal par un manche 2' et est rendu solidaire du tube creux 24 à l'intérieur duquel il est introduit par toute technique appropriée telle que chassage, soudage ou collage. Le manche 2' peut venir de matière avec le cylindre 30 ou être fixé sur celui-ci par exemple par soudage. On peut notamment prévoir qu'à son extrémité proximale, le cylindre 30 présente un diamètre extérieur légèrement supérieur au diamètre intérieur du tube creux 24 de sorte qu'il faille un peu forcer pour introduire ledit cylindre 30 à l'intérieur dudit tube creux 24.

Les figures 3A et 3B permettent de comprendre qu'en fonction du choix du diamètre du cylindre 30, on peut contrôler de manière à la fois simple et précise la largeur d de la fente qui sépare les parties élastiques 28a, 28b du tube creux 24.

Ainsi, conformément aux enseignements de la présente invention, on a recours à des moyens (goupille 22 ou cylindre 30) qui permettent de contrôler l'écartement entre les parties élastiques 16a-16f, respectivement 28a, 28b du tournevis. Ces moyens de contrôle permettent de combler le vide de matière laissé entre les parties élastiques par la ou les fentes pratiquées dans l'extrémité terminale du tournevis selon l'invention et ainsi d'empêcher ces parties élastiques de se rapprocher entre elles et d'être soumises à un phénomène de cisaillement qui peut conduire jusqu'à leur rupture lorsqu'on exerce un couple de serrage au moyen du tournevis.

Il va de soi que la présente invention n'est pas limitée aux modes de réalisation qui viennent d'être décrits et que diverses modifications et variantes simples peuvent être envisagées par l'homme du métier sans sortir du cadre de l'invention tel que défini par les revendications annexées. En particulier, dans le cas du second mode de réalisation, le manche 2' pourrait être réalisé en titane ou en un autre matériau léger pouvant être collé ou soudé sur l'acier trempé dans lequel est réalisé le cylindre 30 pour réaliser un gain en poids du tournevis résultant.

## Revendications

1. Tournevis à usage dentaire ou chirurgical s'étendant sensiblement rectilignement selon un axe de symétrie longitudinale (X-X) et comprenant du côté proximal un manche (2; 2') par lequel le praticien tient le tournevis (1; 1'), ce manche (2; 2') étant prolongé du côté distal par une tige (4; 24, 30) qui présente à son extrémité libre un profil correspondant au profil d'une empreinte pratiquée dans la tête (10) d'une vis (12) à visser, l'extrémité libre de la tige (4; 24, 30) étant séparée par au moins une fente (14a-14c; 26) en au moins deux parties terminales (16a-16f; 28a, 28b) présentant une élasticité, ce tournevis présentant des moyens (22;30) de contrôle de l'écartement de ces deux parties terminales (16a-16f; 28a-28b), ce tournevis (1; 1') étant **caractérisé en ce que** lesdits moyens (22;30) de contrôle de l'écartement sont des moyens (22; 30) de contrôle de l'écartement montés fixes entre les deux parties élastiques (16a-16f; 28a, 28b) qui empêchent ces deux parties élastiques (16a-16f; 28a, 28b) de se rapprocher l'une de l'autre lorsqu'on exerce un couple de serrage au moyen du tournevis (1; 1').

2. Tournevis selon la revendication 1, **caractérisé en ce que** les moyens de contrôle de l'écartement entre les deux parties terminales (16a-16f) de la tige (4) du tournevis (1) comprennent une goupille (22) fixée dans un alésage (20) pratiqué axialement à l'intérieur de la tige (4) du tournevis (1), à partir de l'extrémité libre de celle-ci.

3. Tournevis selon la revendication 2, **caractérisé en ce que** la goupille (22) est rendue solidaire de la tige (4) du tournevis (1) par chassage, collage ou soudage.

4. Tournevis selon la revendication 1, **caractérisé en ce que** le tournevis (1') comprend d'une part un tube creux (24) présentant une extrémité proximale et une extrémité distale, l'extrémité distale du tube creux (24) présentant un profil correspondant au profil d'une empreinte pratiquée dans la tête d'une vis à visser et étant séparée par au moins une fente (26) en au moins deux parties (28a, 28b) présentant une élasticité et, d'autre part, un cylindre (30) disposé coaxialement à l'intérieur du tube creux (24) et s'étendant sensiblement jusqu'au niveau de l'extrémité distale de ce tube (24), le cylindre (30) étant solidaire du tube creux (24) et se prolongeant du côté proximal par un manche (2').

5. Tournevis selon la revendication 4, **caractérisé en ce que** le cylindre (30) peut être un tube creux ou un tube plein.

6. Tournevis selon l'une quelconque des revendications 4 ou 5, **caractérisé en ce que** le cylindre (30) est rendu solidaire du tube creux (24) à l'intérieur duquel il est introduit par chassage, collage ou soudage.

7. Tournevis selon l'une quelconque des revendications 4 à 6, **caractérisé en ce que** le manche (2') vient de matière avec le cylindre (30) ou est fixé sur celui-ci.

8. Tournevis selon la revendication 7, **caractérisé en ce que** le manche (2') est réalisé en titane et est collé ou soudé sur le cylindre (30) réalisé en acier trempé.

9. Tournevis selon l'une quelconque des revendications 1 à 8, **caractérisé en ce que** l'extrémité de la tige (4; 24) du tournevis (1;1') est fendue diamétralement.

10. Tournevis selon la revendication 9. **caractérisé en ce que** l'extrémité de la tige (4; 24) du tournevis (1; 1') est fendue diamétralement selon au moins deux fentes.

## Claims

1. Screwdriver for dental or surgical use extending substantially rectilinearly along a longitudinal axis of symmetry (X-X) and including a handle (2; 2') on the proximal side, via which the practitioner holds the screwdriver (1; 1'), said handle (2: 2') being extended on the distale side by a shaft (4; 24, 30) which has, at the free end thereof, a profile corresponding to the profile of a recess made in the head (10) of a screw (12) to be screwed in, the free end of the shaft (4; 24, 30) being separated by at least one slot (14a-14c; 26) into at least two end portions (16a-16f; 28a, 28b) having elasticity, said screwdriver having means (22; 30) for controlling the distance between said two end portions (16a-16f; 28a, 28b), said screwdriver (1; 1') being **characterized in that** said distance control means (22; 30) comprises fixedly mounted distance control means (22; 30) between the two elastic portions (16a-16f; 28a, 28b) which prevents said two elastic portions (16a-16f; 28a, 28b) from moving close to each other when a tightening torque is exerted by means of the screwdriver (1; 1').

2. Screwdriver according to claim 1, **characterized in that** the means controlling the distance between the two end portions (16a-16f) of the shaft (4) of the screwdriver (1) includes a pin (22) fixed in a bore (20) made axially inside the shaft (4) of the screwdriver (1), starting from the free end thereof.

3. Screwdriver according to claim 2, **characterized in that** the pin (22) is secured to the shaft (4) of the screwdriver (1) by being driven into, bonded or welded to said shaft.

4. Screwdriver according to claim 1, **characterized in that** the screwdriver (1') includes, on the one hand, a hollow tube (24) having a proximal end and a distal end, wherein the profile of the distal end of the hollow tube (24) corresponds to the profile of a recess made in the head of a screw to be screwed in and is separated by at least one slot (26) into at least two portions (28a, 28b) having elasticity, and, on the other hand, a cylinder (30) arranged coaxially inside the hollow tube (24) and extending substantially as far as the distal end of said tube (24), wherein the cylinder (30) is integral with the hollow tube (24) and is extended on the proximal side by a handle (2').

5. Screwdriver according to claim 4, **characterized in that** the cylinder (30) may be a follow tube or a solid tube.

6. Screwdriver according to any of claims 4 or 5, **characterized in that** the cylinder (30) is secured by being driven into, bonded or welded to the hollow tube (24) inside which said cylinder is inserted.

7. Screwdriver according to any of claims 4 to 6, **characterized in that** the handle (2') is in a single-piece with the cylinder (30) or is fixed thereto.

8. Screwdriver according to claim 7, **characterized in that** the handle (2') is made of titanium and is bonded or welded to the cylinder (30), which is made of hardened steel.

9. Screwdriver according to any of claims 1 to 8, **characterized in that** the end of the shaft (4; 24) of the screwdriver (1; 1') is split diametrally.

10. Screwdriver according to claim 9, **characterized in that** the end of the shaft (4; 24) of the screwdriver (1; 1') is split diametrally into at least two slots.

## Patentansprüche

1. Schraubendreher zur zahnärztlichen oder chirurgischen Nutzung, der sich im Wesentlichen geradlinig längs einer longitudinalen Symmetrieachse (X-X) erstreckt und auf der proximalen Seite einen Griff (2; 2'), mit dem der Arzt den Schraubendreher (1; 1') hält, umfasst, wobei dieser Griff (2; 2') auf der distalen Seite durch einen Stift (4; 24, 30) verlängert ist, der an seinem freien Ende ein Profil aufweist, das dem Profil einer im Kopf (10) einer zu schraubenden Schraube (12) ausgebildeten Prägung entspricht, wobei das freie Ende des Stifts (4; 24, 30) durch wenigstens einen Schlitz (14a-14c; 26) in wenigstens zwei Endteile (16a-16f; 28a, 28b) getrennt ist, die eine Elastizität aufweisen, wobei dieser Schraubendreher Mittel (22; 30) für die Steuerung des Abstands dieser zwei Endteile (16a-16f; 28a-28b) aufweist, wobei dieser Schraubendreher (1; 1') **dadurch gekennzeichnet ist, dass** diese Mittel (22; 30) für die Steuerung des Abstands Mittel (22; 30) für die Steuerung des Abstands sind, die zwischen den zwei elastischen Teilen (16a-16f; 28a, 28b) fest montiert sind und verhindern, dass sich diese zwei elastischen Teile (16a-16f; 28a, 28b) einander annähern, wenn mittels des Schraubendrehers (1; 1') ein Anziehdrehmornent ausgeübt wird.

2. Schraubendreher nach Anspruch 1, **dadurch gekennzeichnet, dass** die Mittel für die Steuerung des Abstands zwischen den zwei Endteilen (10a-10f) des Stifts (4) des Schraubendrehers (1) einen Zapfen (22) aufweisen, der in einer Bohrung (20) befestigt ist, die in dem Stift (4) des Schraubendrehers (1) ausgehend von seinem freien Ende axial ausgebildet ist.

3. Schraubendreher nach Anspruch 2, **dadurch gekennzeichnet, dass** der Zapfen (22) mit dem Stift (4) des Schraubendrehers (1) durch Eintreiben, Kleben oder Schweißen fest verbunden ist.

4. Schraubendreher nach Anspruch 1, **dadurch gekennzeichnet, dass** der Schraubendreher (1') einerseits ein Hohlrohr (24), das ein proximales Ende und ein distales Ende aufweist, wobei das distale Ende des Hohlrohrs (24) ein Profil aufweist, das dem Profil einer im Kopf einer zu schraubenden Schraube ausgebildeten Prägung entspricht, und durch wenigstens einen Schlitz (26) in wenigstens zwei Teile (28a, 28b) getrennt ist, die eine Elastizität aufweisen, und andererseits einen Zylinder (30), der koaxial in dem Hohlrohr (24) angeordnet ist und sich im Wesentlichen bis auf die Höhe des distalen Endes dieses Rohrs (24) erstreckt, umfasst, wobei der Zylinder (30) mit dem Hohlrohr (24) verbunden ist und auf der proximalen Seite durch einen Griff (2') verlängert ist.

5. Schraubendreher nach Anspruch 4, **dadurch gekennzeichnet, dass** der Zylinder (30) ein Hohlrohr oder ein Vollrohr sein kann.

6. Schraubendreher nach einem der Ansprüche 4 oder 5, **dadurch gekennzeichnet, dass** der Zylinder (30) mit dem Hohlrohr (24), in das er durch Eintreiben, Kleben oder Verschweißten eingeführt ist, fest verbunden ist.

7. Schraubendreher nach einem der Ansprüche 4 bis 6, **dadurch gekennzeichnet, dass** der Griff (2') mit dem Zylinder (30) einteilig ausgebildet ist oder an diesem befestigt ist.

8. Schraubendreher nach Anspruch 7, **dadurch gekennzeichnet, dass** der Griff (2') aus Titan hergestellt ist und an den aus gehärtetem Stahl her gestellten Zylinder (30) geklebt oder geschweißt ist.

9. Schraubendreher nach einem der Ansprüche 1 bis 8, **dadurch gekennzeichnet, dass** das Ende des Stifts (4; 24) des Schraubendrehers (1; 1') diametral geschlitzt ist.

10. Schraubendreher nach Anspruch 9, **dadurch gekennzeichnet, dass** das Ende des Stifts (4; 24) des Schraubendrehers (1; 1') mit wenigstens zwei Schlitzen diametral geschlitzt ist.
